# EUROPÄISCHE PATENTSCHRIFT

(11) **EP 0 330 924 B1**
(45) Veröffentlichungstag und Bekanntmachung des Hinweises auf die Patenterteilung: **24.05.1995**
(21) Anmeldenummer: 89102704.7
(22) Anmeldetag: 17.02.1989
(51) Int. Cl.: A61K 31/44

(54) **Verwendung von 1,4-Dihydropyridin-Derivaten in der Behandlung von Alkoholsucht**
Use of 1,4-dihydropyridine derivatives in the treatment of alcohol addiction
Utilisation des dérivés de la 1,4-dihydropyridine dans le traitement de la dépendance à l'alcool

(30) Priorität: 27.02.1988 DE 3806277
(43) Veröffentlichungstag der Anmeldung: 06.09.1989
(73) Patentinhaber: Troponwerke GmbH & Co. KG, D-51063 Köln (DE)
(72) Erfinder: Opitz, Klaus, Prof. Dr., D-4400 Münster (DE); Traber, Jörg, Dr., D-5204 Lohmar 21 (DE)
(74) Vertreter: Mann, Volker, Dr.

(56) Entgegenhaltungen:
- EP-A- 0 204 951
- DE-A- 3 741 414
- M. WINDHOLZ et al.: "The Merck Index", Ausgabe 10, 1983, Seite 569, Merck & Co., Inc., Rahway, NJ, US
- ANNALS OF THE NEW YORK ACADEMY OF SCIENCES, Band 492, 1987, Seiten 156-170, US; M.J. HUDSPITH et al.: "Dihydropyridine-sensitive Ca2+ channels and inositol phospholipid metabolism in ethanol physical dependence"
- BRITISH JOURNAL OF PHARMACOLOGY, Band 92, Supplement, 9. - 11. September 1987, Seite 606P, GB; H.J. LITTLE et al.: "Lack of tolerance to ethanol after concurrent administration of nitrendipine"
- NEUROPHARMACOLOGY, Band 26, Nr. 2/3, Februar/März 1987, Seiten 275-279; S. DOLIN et al.: "Increased dihydropyridine-sensitive calcium channels in rat brain may underlie ethanol physical dependence"
- BRITISH JOURNAL OF PHARMACOLOGY, Band 90, Supplement, 17. - 19. Dezember 1986, Seite 210P, GB; S.J. DOLIN et al.: "Dihydropyridine-sensitive calcium channels in rat brain are increased in ethanol physical dependence"
- JOURNAL OF NEURAL TRANSMISSION, Band 74, Nr. 3, 1988, Seiten 181-193; J.A. ENGEL et al.: "Biochemical and behavioral evidence for an interaction between ethanol and calcium channel antagonists"
- ANNALS OF THE NEW YORK ACADEMY OF SCIENCES, Band 560, 1989, Seiten 465-466, US, CONFERENCE PAPER, London, 18. - 20. Juli 1988; H.J. LITTLE et al.: "Possible role of calcium channels in ethanol tolerance and dependence"
- LIFE SCIENCES, Band 39, 1986, Seiten 2059-2965, US; H.J. LITTLE et al.: "Calcium channel antagonists decrease the ethanol withdrawal syndrome"
- IDEM
- Dr. W. FORTH et al.: "Allgemeine und spezielle Pharmakologie und Toxikologie, Auflage 4, 1983, Seiten 510-513, Bibliographisches Institut, Mannheim, DE
- D8:MSD-Manual,1975, S. 1594 - 1600
- D9: Pschyrembel, 1977, S. 1178
- D 10: Roche Lexikon Medizin, 1987, S.1648 und literature

## Beschreibung

Die Erfindung betrifft die Verwendung von calcium-antagonistisch wirksamen Dihydropyridinen zur Herstellung von Arzneimitteln für die Behandlung von Alkoholsucht.

Dihydropyridine mit kalziumantagonistischer Wirkung sind bekannt (GB 1 173 862, GB 1 358 951, US 4 256 749 und US 4 264 611). Für diese Dihydropyridine sind bereits eine Reihe von pharmakologischen Wirkungen beschrieben wie z.B. Coronarwirkung, Blutdruckwirkung, diuretische Wirkung oder antiischämische Wirkung im cerebralen Bereich.

Aus Life Sciences 39, 2059 bis 2065 (1986), ist bekannt, daß bei alkoholabhängigen Ratten, die mit den 1,4-Dihydropyridinen Nitrendipin und Nimodipin behandelt werden, das Auftreten von Entzugserscheinungen weitgehend verhindert werden kann. Entzugserscheinungen äußern sich bei Alkoholikern durch Übelkeit, Erbrechen, Durchfälle, Krampfanfälle, Schlaflosigkeit und Delirien (Roche Lexikon, Medizin, 8 (1984)).

Es wurde die Verwendung von calcium-antagonistisch wirksamen Dihydropyridinen der allgemeinen Formel (I) gefunden, 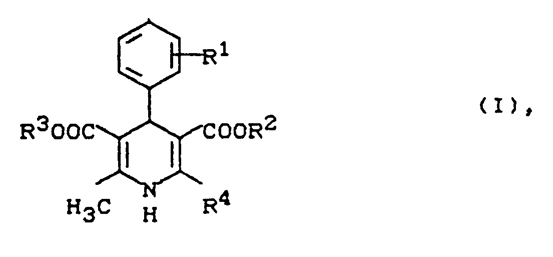
in welcher
- R¹: für ein oder zwei gleiche oder verschiedene Substituenten aus der Gruppe Nitro, Halogen, Trifluormethyl oder OCHF₂ steht,
- R² und R³: gleich oder verschieden sind und jeweils für Alkyl mit 1 bis 12 Kohlenstoffatomen stehen, welches gegebenenfalls substituiert ist durch Alkoxy mit 1 bis 4 C-Atomen, Hydroxy, Halogen oder N-Methyl-N-Benzylamino und
- R⁴: für Cyano oder Alkyl mit 1 bis 4 Kohlenstoffatomen steht, welches gegebenenfalls substituiert ist durch Hydroxy oder Halogen,
zur Herstellung von Arzneimitteln zur Behandlung von Alkoholsucht.

Unter Alkoholsucht (craving) versteht man das zwanghafte Angewiesensein auf Alkoholkonsum. Der Alkoholsüchtige erkennt die Folgen seines Verhaltens aber setzt den Alkoholgenuß trotz Einsicht fort (Unfähigkeit zur Abstinenz).

Überraschenderweise führt eine Behandlung mit calcium-antagonistisch wirksamem Dihydropyridin zu einer nachhaltigen Aufhebung der Suchterscheinungen.

Im Rahmen der Formel (I) haben die Substituenten im allgemeinen die folgende Bedeutung:

Halogen kann für Fluor, Chlor, Brom und Jod, insbesondere für Fluor und Chlor, stehen.

Alkyl (R² und R³) kann ein geradkettiger oder verzweigter Kohlenwasserstoffrest mit 1 bis 12, bevorzugt 1 bis 6, Kohlenstoffatomen sein. Bevorzugt werden Methyl, Ethyl, Propyl, iso-Propyl, Butyl, iso-Butyl, Pentyl, iso-Pentyl, Hexyl und iso-Hexyl.

Der Kohlenwasserstoffrest in den Alkyl- oder Alkoxygruppen kann geradkettig oder verzweigt sein und 1 bis 4 Kohlenstoffatome enthalten. Bevorzugt werden als Alkylgruppen Methyl, Ethyl, Propyl, iso-Propyl, Butyl und iso-Butyl und als Alkoxygruppen Methoxy, Ethoxy, Propoxy, iso-Propoxy, Butoxy und iso-Butoxy genannt.

Von besonderer Bedeutung sind die calcium-antagonistisch wirksamen Dihydropyridine aus der Gruppe Nifedipin, Niludipin, Nisoldipin, Nitrendipin, Nimodipin, Felodipin, Nicardipin.

Entsprechende erfindungsgemäße Arzneimittel sind gekennzeichnet durch einen Gehalt an calcium-antagonistisch wirksamen Dihydropyridinen der Formel (I).

Die Herstellung der calcium-antagonistisch wirksamen Dihydropyridinen ist an sich bekannt und kann beispielsweise durch Umsetzung von entsprechenden Ylidenderivaten mit Enaminen erfolgen (DE-A 33 12 283).

Die erfindungsgemäßen Arzneimittel enthalten im allgemeinen 1 bis 15 Gew.-%, bevorzugt 5 bis 10 Gew.-% an calcium-antagonistisch wirksamen Dihydropyridinen, bezogen auf die Zubereitung.

Es ist selbstverständlich möglich, daß die erfindungsgemäßen Arzneimittel weitere an sich bekannte Wirkstoffe enthalten.

Die erfindungsgemäßen Arzneimittel können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/ Sesamöl), Alkohole (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin-Sulfitablaugen, Methylzellulose, Stärke und Polyvinylpyrrolidon) und Gleitmittel (z.B. Magnesiumstearat, Talkum, Stearinsäure und Natriumlaurysulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie stärke, vorzugsweise Kartoffelstärke, Gelatine und dergleichen enthalten. Weiterhin können Gleitmittel, wie Magnesiumstearat, Natriumlaurysulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fälllen ausreichend sein, mit weniger als der vorgenannten Mindestmenge auszukommen, wenn in anderen Fällen die genannte obere Grenze überschritten werden muß. Im Falle der Applikation größerer Mengen kann es empfehlenswert sein, diese in mehreren Einzelgaben über den Tag zu verteilen.

Ein befriedigendes Mittel zur medikamentösen Behandlung der Alkoholsucht ist nicht bekannt. Die Enzymhemmstoffe Disulfiram und Nitrefazol bewirken eine unangenehme Reaktion, wenn der damit behandelte Alkoholiker Alkohol trinkt, so daß dieser den Alkohol trotz bestehenden Verlangens absetzt.

Im Gegensatz zu diesen Substanzen hemmen die calcium-antagonistisch wirksamen Dihydropyridine erfindungsgemäß den freiwilligen Alkoholkonsum Alkoholabhängiger. Insbesondere kann der Gebrauch von calcium-antagonistisch wirksamen Dihydropyridinen das Rückfälligwerden verhindern.

### Beispiel

### Bestimmung der Wirksamkeit

Ethanol-präferente Ratten werden unter standardisierten Bedingungen (12-Stunden-Hell-Dunkel-Rhythmus, 23 ± 1°C) einzeln in großen Käfigen gehalten. Den Tieren stehen Zuchtfutter, Trinkwasser und Ethanol 10 Vol-% in unbeschränkten Mengen zur Verfügung, jedoch nur während der Dunkelphase von 20.00 bis 08.00 Uhr. Die zu untersuchenden Substanzen bzw. das Lösungsmittel (2 ml Cremophor® EL, 0,3 ml 1,2-Propandiol, aqua dest. ad 10 ml) werden einmal oral zugeführt (2 ml/kg, Schlundsonde), und zwar 30 bis 20 Min. vor Beginn der Dunkelphase. Die Futtergefäße und die Trinkflaschen werden jeden Morgen gewogen und die verbrauchten Mengen festgestellt. Als Maß der Präferenz gilt die getrunkene Alkoholmenge (10 Vol-%) im Prozent der Gesamtflüssigkeitsaufnahme. Die nach Verabreichung einer Prüfsubstanz gemessenen Verbräuche werden mit den mittleren Verbräuchen an den drei vorangegangenen Tagen (Vorperiode) verglichen. Angegeben in Tabelle 1 ist die jeweilige Änderung der Gesamtflüssigkeitsaufnahme und die Änderung des in Gramm/kg Körpergewicht gemessenen absoluten Alkoholkonsums in Prozent der während der jeweils dreitägigen Vorperiode ermittelten Durchschnittswerte. Statistische Berechnungen erfolgen nach dem Student's t-Test für gepaarte Werte.

**Tabelle 1**

| Ingestives Verhalten von acht männlichen ethanol-präferenten Ratten | | |
|---|---|---|
| Nimodipin-Dosis (mg/kg p.o.) | Änderung der Gesamtflüssigkeitsaufnahme (%) | Änderung des absoluten Alkoholkonsums (%) |
| 0 (Lösungsmittel) | + 6,9 ns | + 8,4 ns |
| 5 | + 1,7 ns | - 26,2 * |
| 10 | - 1,8 ns | - 44,0 ** |

| | | |
|---|---|---|
| * p <0,05 | | |
| ** p <0,001 | | |
| ns nicht signifikant | | |

Die Daten zeigen für Nimodipin eine starke, hochsignifikante Abnahme des absoluten Alkoholkonsums. Demgemäß nimmt nach Gabe von Nimodipin die Präferenz der Ratten für Alkohol stark ab. Die Gesamtflüssigkeitsaufnahme wird nicht signifikant verändert.

## Patentansprüche

1. Verwendung von kalziumantagonistisch wirksamen Dihydropyridinen der allgemeinen Formel (I) 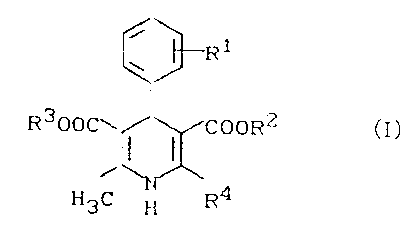 in welcher
R¹ für ein oder zwei gleiche oder verschiedene Substituenten aus der Gruppe Nitro, Halogen, Trifluormethyl oder OCHF₂ steht,
R² und R³ gleich oder verschieden sind und jeweils für Alkyl mit 1 bis 12 Kohlenstoffatomen stehen, welches gegebenenfalls substituiert ist durch Alkoxy mit 1 bis 4 C-Atomen, Hydroxy, Halogen oder N-Methyl-N-Benzylamino und
R⁴ für Cyano oder Alkyl mit 1 bis 4 Kohlenstoffatomen steht, welches gegebenenfalls substituiert ist durch Hydroxy oder Halogen,
zur Herstellung von Arzneimitteln zur Behandlung der Alkoholsucht.

2. Verwendung von calciumantagonistisch wirksamen Dihydropyridinen der allgemeinen Formel nach Anspruch 1,
worin
R¹ - für ein oder zwei gleiche oder verschiedene Substituenten aus der Gruppe Nitro, Fluor, Chlor, Trifluormethyl oder OCHF₂ steht,
R² und R³ gleich oder verschieden sind und jeweils für Alkyl mit 1 bis 4 Kohlenstoffatomen stehen, welches gegebenenfalls substituiert ist durch Alkoxy mit 1 bis 4 Kohlenstoffatomen, Hydroxy, Fluor, Chlor oder N-Methyl-N-benzylamino und
R⁴ - für Cyano oder Alkyl mit 1 bis 4 Kohlenstoffatomen steht, welches gegebenenfalls substituiert ist durch Hydroxy, Fluor oder Chlor
zur Herstellung von Arzneimitteln zur Behandlung der Alkoholsucht.

3. Verwendung nach Anspruch 1, dadurch gekennzeichnet, daß als 1,4-Dihydropyridin-Derivat Nimodipin, Niludipin, Nisoldipin, Nitrendipin, Nimodipin, Felodipin oder Nicardipin eingesetzt wird.

## Claims

1. Use of dihydropyridines with a calcium-antagonistic action of the general formula (I) 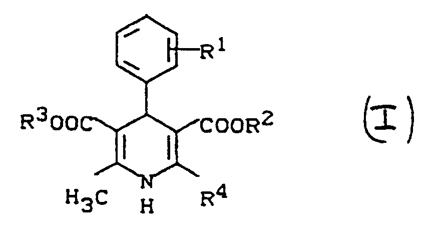 in which
R¹ represents one or two identical or different substituents from the group comprising nitro, halogen, trifluoromethyl or OCHF₂,
R² and R³ are identical or different and each represents alkyl with 1 to 12 carbon atoms, which is optionally substituted by alkoxy with 1 to 4 C atoms, hydroxyl, halogen or N-methyl-N-benzylamino and
R⁴ represents cyano or alkyl with 1 to 4 carbon atoms, which is optionally substituted by hydroxyl or halogen,
for the preparation of medicaments for the treatment of alcohol addiction.

2. Use of dihydropyridines with a calcium-antagonistic action of the general formula (I) according to Claim 1,
in which
R¹ represents one or two identical or different substituents from the group comprising nitro, fluorine, chlorine, trifluoromethyl or OCHF₂,
R² and R³ are identical or different and each represents alkyl with 1 to 12 carbon atoms, which is optionally substituted by alkoxy with 1 to 4 carbon atoms, hydroxyl, fluorine, chlorine or N-methyl-N-benzyl-amino and
R⁴ represents cyano or alkyl with 1 to 4 carbon atoms, which is optionally substituted by hydroxyl, fluorine or chlorine
for the preparation of medicaments for the treatment of alcohol addiction.

3. Use according to Claim 1, characterized in that nimodipine, niludipine, nisoldipine, nitrendipine, nimodipine, felodipine or nicardipine is used as the 1,4-dihydropyridine derivative.

## Revendications

1. Utilisation de dihydropyridines antagonistes du calcium, de formule générale (I) 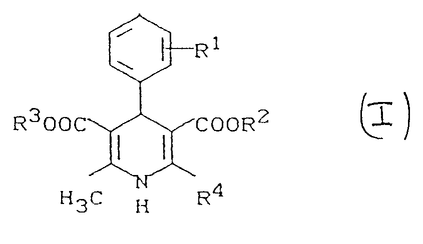 dans laquelle
R¹ représente un ou deux substituants identiques ou différents, du groupe nitro, halogéno, trifluorométhyle ou OCHF₂,
R² et R³ sont identiques ou différents et chacun d'eux représente un groupe alkyle ayant 1 à 12 atomes de carbone, qui est substitué le cas échéant par un radical alkoxy ayant 1 à 4 atomes de carbone, hydroxy, halogéno ou N-méthyl-N-benzylamino et
R⁴ est un groupe cyano ou un groupe alkyle ayant 1 à 4 atomes de carbone, qui est substitué le cas échéant par un radical hydroxy ou un halogène,
pour la préparation de médicaments destinés au traitement de la dépendance à l'égard de l'alcool.

2. Utilisation de dihydropyridines antagonistes du calcium de formule générale suivant la revendication 1,
dans laquelle
R¹ représente un ou deux substituants identiques ou différents du groupe nitro, fluoro, chloro, trifluorométhyle ou OCHF₂,
R² et R³ sont identiques ou différents et représentent chacun un groupe alkyle ayant 1 à 4 atomes de carbone, qui est substitué le cas échéant par un radical alkoxy ayant 1 à 4 atomes de carbone, hydroxy, fluoro, chloro ou N-méthyl-N-benzylamino et
R⁴ est un groupe cyano ou un groupe alkyle ayant 1 à 4 atomes de carbone, qui est substitué le cas échéant par un radical hydroxy, fluoro ou chloro,
pour la préparation de médicaments destinés au traitement de la dépendance à l'égard de l'alcool.

3. Utilisation suivant la revendication 1, caractérisée en ce que le dérivé de 1,4-dihydropyridine utilisé est la nimodipine, la niludipine, la nisoldipine, la nitréndipine, la nimodipine, la félodipine ou la nicardipine.
